(19) Europäisches Patentamt
European Patent Office
Office européen des brevets

(11) Numéro de publication : **0 409 670 B1**

(12) **FASCICULE DE BREVET EUROPEEN**

(45) Date de publication du fascicule du brevet : **03.03.93 Bulletin 93/09**

(51) Int. Cl.[5] : **A61K 7/18**

(21) Numéro de dépôt : **90401327.3**

(22) Date de dépôt : **18.05.90**

(54) **Dentifrices anti-caries contenant un fluorure de polymère polycationique.**

(30) Priorité : **18.05.89 FR 8906500**

(43) Date de publication de la demande : **23.01.91 Bulletin 91/04**

(45) Mention de la délivrance du brevet : **03.03.93 Bulletin 93/09**

(84) Etats contractants désignés : **AT BE CH DE DK ES FR GB GR IT LI NL SE**

(56) Documents cités :
**EP-A- 0 200 903**
**EP-A- 0 228 209**

(73) Titulaire : **L'OREAL**
**14, Rue Royale**
**F-75008 Paris (FR)**

(72) Inventeur : **Grollier, Jean-François**
**16 Bis, Boulevard Morland**
**F-75004 Paris (FR)**

(74) Mandataire : **Tonnellier, Jean-Claude et al**
**Cabinet Nony & Cie. 29, rue Cambacérès**
**F-75008 Paris (FR)**

EP 0 409 670 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet de nouvelles compositions dentrifrices à action anti-caries, contenant un fluorure de polymère polycationique.

Dans la suite de la description on entend par composition dentifrice toute composition solide ou liquide destinée à l'hygiène buccale, telle que pâte dentifrice, gomme à mâcher, poudre ou bain de bouche.

On sait que l'utilisation des produits de nettoyage des dents, et en particulier des compositions dentifrices, a pour but essentiel, en ce qui concerne la prévention des caries, d'éliminer la plaque dentaire ou d'empêcher sa formation. Cette plaque dentaire, qui est constituée de résidus d'aliments transformés par le métabolisme bactérien, est un dépôt qui durcit et adhère fortement aux dents s'il n'est pas enlevé rapidement. La plaque dentaire installée favorise la multiplication de bactéries qui transforment les sucres en produits acides. Ces produits acides attaquent l'émail des dents et les déminéralisent. En l'absence de traitement, les bactéries et leurs sécrétions acides peuvent progressivement atteindre la pulpe de la dent, allant jusqu'à provoquer sa destruction.

On sait également que la plaque dentaire se déposant sur les dents et sur les gencives dans leur zone de contact joue un rôle important dans diverses affections gingivales.

Il est connu que les produits dentifrices usuels qui contiennent principalement des particules abrasives (agents de polissage) et des tensio-actifs (agents nettoyants et moussants) ne permettent pas une élimination suffisante de la plaque dentaire et la prévention des caries.

Pour lutter plus efficacement contre les caries, on a proposé l'utilisation de divers produits bactéricides capables de détruire ou de limiter dans de fortes proportions les populations de bactéries responsables de la formation de la plaque dentaire.

Il faut toutefois noter que l'utilisation d'agents bactéricides pose des problèmes de formulation, car il convient de trouver des agents bactéricides qui sont compatibles à la fois avec l'agent abrasif et avec l'agent tensio-actif. Ces difficultés de formulation sont suffisamment importantes pour que des chercheurs aient pu recommander l'utilisation successive de deux compositions séparées, l'une contenant l'agent tensio-actif et l'autre contenant l'agent bactéricide ; voir la demande de brevet français n°75.26219 publiée sous le n°2.282.861.

On a également cherché à prévenir les caries en essayant de mettre à profit les propriétés des ions fluorure utilisés à faible dose. Il est généralement admis que les ions fluorure ont pour effet de transformer l'hydroxyapatite de l'émail dentaire en fluoroapatite, ce qui accroît la cristallinité et la dureté de l'émail et le rend ainsi plus résistant à l'attaque des acides.

On a préconisé l'utilisation de fluorures métalliques comme le fluorure de sodium et le fluorure stanneux dans les compositions de dentrifrice. Toutefois ces fluorures présentent l'inconvénient d'une faible compatibilité avec les agents de polissage minéraux usuels contenus dans les pâtes dentifrices, et en particulier avec l'alumine et le carbonate de calcium. Les interactions entre ces fluorures et les agents de polissage conduisent notamment à une diminution de la teneur en ions fluorure libres et donc à une baisse d'efficacité.

Pour remédier à ces inconvénients, on a proposé l'utilisation de monofluorophosphate qui est davantage compatible avec les agents de polissage. Toutefois, même avec l'utilisation de produits dentifrices fortement dosés en fluorophosphate, la fixation des ions fluorure sur les dents demeure assez faible, compte tenu du temps de contact limité entre la composition dentifrice et la dent.

Cependant le monofluorophosphate n'agit pas directement sur la cause première des caries, à savoir les bactéries. C'est la raison pour laquelle, en vue d'accroître l'efficacité des ions fluorure, on leur a souvent adjoint des agents antibactériens antiplaque.

On a également proposé d'appliquer sur les dents des matériaux fluides capables de durcir rapidement pour former un revêtement de résine protectrice pouvant jouer le rôle d'échangeuse d'ions fluorure. Toutefois de telles compositions fluides durcissables ne peuvent être employées en pratique que sur des dents déjà atteintes de caries et ne peuvent pas être utilisées pour les soins d'hygiène courante dans des compositions dentifrices. En outre, la nécessité d'appliquer une composition non durcie présente divers inconvénients dus notamment à la toxicité des composés monomères et éventuellement des initiateurs de polymérisation dont la présence est nécessaire ; voir l'article de H.R. RAWLS et P.F. ZIMMERMAN, Caries Res. 17 : 32-43 (1983).

On a maintenant découvert que les fluorures de certains polymères polycationiques comprenant des motifs récurrents qui contiennent un azote quaternisé peuvent être incorporés dans les compositions dentifrices comme agents anti-caries.

On a également découvert que l'utilisation de tels fluorures est compatible avec l'utilisation des agents de polissage minéraux pouvant être présents dans ces compositions.

On pouvait en effet redouter que ces polymères polycationiques soient adsorbés, et en conséquence inactivés, sur les agents de polissage qui sont utilisés à l'état finement divisé et qui présentent donc une grande

surface de contact. Or les recherches de la Société demanderesse ont permis de découvrir que dans les compositions dentifrices de l'invention, les fluorures de polymères polycationiques, malgré la présence d'agents de polissage minéraux finement divisés, restent actifs et permettent une protection contre la déminéralisation des dents.

On a également découvert que les fluorures de polymères polycationiques utilisés selon l'invention sont compatibles avec les agents tensio-actifs usuels utilisés dans les dentifrices, y compris avec les tensioactifs anioniques.

D'autre part, ces fluorures de polymères polycationiques possèdent une activité bactéricide qui renforce leur action contre la plaque dentaire et contre les caries.

En outre, on a remarqué que la présence des fluorures de polymères polycationiques, dans les compositions dentifrices de l'invention, retardé le dessèchement à l'air de ces compositions et facilite leur préparation, en particulier leur homogénéisation.

La présente invention a donc pour objet une composition dentifrice, contenant les ingrédients usuels d'une telle composition, caractérisée par le fait qu'elle contient, à titre d'agent anti-caries, au moins un fluorure d'un polymère polycationique comprenant des motifs qui contiennent au moins un azote quaternisé faisant partie de la macrochaîne, ledit fluorure de polymère polycationique étant soluble dans l'eau.

Les ingrédients usuels présents dans les compositions dentifrices, ainsi que les modes de préparation de ces compositions, sont bien connus et sont décrits par exemple dans les ouvrages suivants : Handbook of Cosmetic Science, H.W. Hibbott Ed., Pergamon Press (Oxford, Londres, New-York, Paris), et Harry's Cosmeticology, 6è Edition, Leonard Hill Books (Londres).

Il convient de noter que,par convention, on désigne dans la présente demande par "fluorure de polymères polycationiques" non seulement des polymères polycationiques dont les charges cationiques sont équilibrées totalement par des ions fluorure, mais aussi des polymères polycationiques dont au moins 5% des charges cationiques sont équilibrées par des anions fluorure. Bien entendu, les autres charges cationiques sont équilibrées par d'autres anions, en particulier des ions chlorure et/ou des ions bromure.

Dans les compositions de l'invention, la concentration en polymère polycationique peut varier dans des proportions pouvant aller de 0,1 à 5% en poids par rapport au poids total de la composition, et de préférence de 0,5 à 5% en poids.

De préférence la proportion d'ions fluorure est telle que la concentration en ions fluorure ne dépasse pas 1500 ppm par rapport au poids total de la composition.

Il est bien entendu possible d'utiliser dans les compositions de l'invention des mélanges de fluorures de polymères polycationiques. Il est également possible d'y adjoindre d'autres dérivés fluorés comme par exemple des fluorures de métaux alcalins ou des monofluorophosphates alcalins.

Les fluorures de polymères polycationiques sont préparés selon les méthodes usuelles, par exemple au départ des autres halogénures (chlorures, bromures), notamment par échange d'ions.

Bien entendu, les polymères polycationiques utilisés dans les compositions de l'invention sont des polymères non toxiques.

Parmi les polymères polycationiques dont les fluorures sont utilisables dans les compositions de la présente invention, on citera notamment ceux dans lesquels chaque atome d'azote quaternisé de la macrochaîne possède deux substituants latéraux choisis parmi des groupements aliphatiques, hydroxyaliphatiques, alicycliques ou arylaliphatiques substitués ou non, contenant au maximum 20 atomes de carbone, ou bien les deux substituants latéraux attachés à un même atome d'azote constituent, ensemble, avec celui-ci, un cycle pouvant contenir un second hétéroatome autre que l'azote, ou bien encore deux substituants latéraux attachés à deux atomes d'azote quaternisés consécutifs représentent ensemble un groupement divalent. En outre, dans ces polymères, deux atomes d'azote quaternisés consécutifs sont bien entendu reliés par un groupement divalent formant, avec lesdits atomes d' azote quaternisés, la macrochaîne, ledit groupement divalent étant notamment un groupement alcoylène ou arylène éventuellement substitué et pouvant comporter des hétéroatomes.

Parmi ces polymères polycationiques, on citera en particulier ceux dont la macrochaîne est constituée par la succession de deux desdits groupements divalents, présents alternativement dans la chaîne, chacun entre deux atomes d'azote quaternisés consécutifs, ces deux radicaux divalents pouvant éventuellement être identiques. On sait que de tels polymères forment une classe connue de polymères polycationiques comprenant notamment les ionènes.

Dans la présente demande, sauf précisions contraires, les groupements aliphatiques, y compris les groupements alkyle, et les groupements divalents correspondants, y compris les alkylènes, ont le plus souvent de 1 à 12 atomes de carbone; les groupements aryle (ou arylène) ont le plus souvent 6 à 20 atomes de carbone et sont généralement des groupements dérivés du phényle (ou phénylène), du naphtyle (ou naphtylène), ou encore les groupements arylène sont des groupements bis-phénylène.

Parmi les polymères polycationiques du type ionène, on peut citer en particulier, à titre d'exemple non limitatif, ceux qui comprennent, ou sont constitués par des motifs récurrents de formule I,

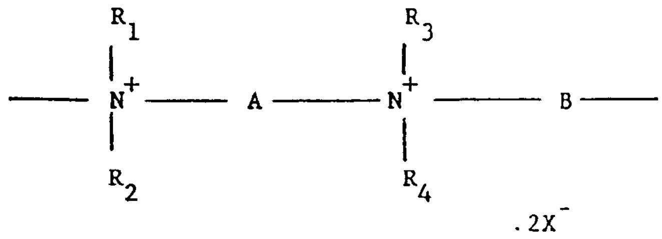

(I)

dans laquelle :

$R_1$, $R_2$, $R_3$ et $R_4$, identiques ou différents, représentent un groupement aliphatique, alicyclique ou arylaliphatique, éventuellement substitué, contenant au maximum 20 atomes de carbone, ou un groupement hydroxyaliphatique ayant 1 à 8 atomes de carbone; ou bien deux substituants ($R_1$ et $R_2$) et/ou ($R_3$ et $R_4$) attachés à un même atome d'azote peuvent représenter ensemble un groupement divalent formant, avec l'atome d'azote auquel ils sont rattachés, un hétérocycle pouvant contenir un ou plusieurs hétéroatomes autres que l'azote; ou bien les couples ($R_1$ et $R_3$) et/ou ($R_2$ et $R_4$) forment ensemble un groupement divalent reliant les deux atomes d'azote consécutifs, représentés dans le motif de formule I, auxquels ils sont respectivement rattachés ;

A et B, identiques ou différents, représentent un groupement alkylène linéaire ou ramifié ayant 2 à 20 atomes de carbone, éventuellement insaturé, ledit groupement alkylène pouvant être substitué et/ou interrompu par un ou plusieurs hétéroatomes, et/ou par un ou plusieurs groupements hétéroatomiques et/ou par un ou plusieurs cycles aliphatiques ou aromatiques ou par un ou plusieurs hétérocycles; un groupement cycloalkylène, éventuellement substitué et/ou comportant éventuellement des doubles liaisons, pouvant contenir jusqu'à 20 atomes de carbone; un ou plusieurs groupements arylène ayant 6 à 20 atomes de carbone, lesdits groupements arylène étant éventuellement substitués, et/ou séparé par un ou plusieurs hétéroatomes et/ou par un ou plusieurs groupements hétéroatomiques et/ou par un ou plusieurs groupements alkylène et/ou par un ou plusieurs cycles aliphatiques et/ou par un ou plusieurs hétérocycles ;

et $X^-$ représente un anion,

étant entendu que, dans une composition selon l'invention, au moins 5%, en nombre, des anions présents dans le polymère polycationique ayant des motifs de formule I sont des ions fluorure.

Dans les motifs de formule I :

- $R_1$ et/ou $R_3$ représentent notamment un groupement alkyle ou hydroxyalkyle ayant 1 à 6 atomes de carbone;
- $R_2$ et/ou $R_4$ représentent notamment un groupement alkyle ou hydroxyalkyle ayant par exemple 1 à 16 atomes de carbone, ou un groupement cycloalkylalkyle (le cycloalkyle ayant 5 ou 6 chaînons) ou aralkyle (notamment phénylalkyle) dont l'alkyle a par exemple 1 à 3 atomes de carbone, ou un groupement cycloalkyle à 5 ou 6 chaînons;
- ou un ou plusieurs groupements $R_1$, $R_2$, $R_3$, $R_4$ représentent un groupement

$$-CH_2CH(R'_1)(R'_2)$$

dans lequel $R'_1$ désigne -H ou un groupement alkyle inférieur et $R'_2$ désigne l'un des groupements suivants :

$$-CN\ ,-CO-OR'_3\ ,-CO-R'_3\ ,-CO-N(R'_4)_2,-\ CO-O-R'_5-Y$$

et

$$CO-NH-R'_5-Y$$

$R'_3$ étant un groupement alkyle inférieur, $R'_6$ étant -H ou un groupement alkyle inférieur, $R'_5$ étant un alkylène inférieur, et Y étant un groupement ammonium quaternaire;

- ou, lorsque deux restes ($R_1$ et $R_2$), ou ($R_3$ et $R_4$), attachés à un même atome d'azote, constituent avec celui-ci un hétérocycle, ils peuvent représenter ensemble notamment un groupement polyméthylène ayant 2 à 5 atomes de carbone éventuellement substitué et/ou interrompu par un hétéroatome d'oxygène;
- lorsque le couple ($R_1$, $R_3$) et/ou le couple ($R_2$, $R_4$) forment ensemble un groupement divalent, ledit groupement divalent est notamment un groupement hydrocarboné, éventuellement insaturé, ayant 2 à 10 atomes de carbone; par exemple les couples ($R_1$, $R_3$) et/ou ($R_2$, $R_4$) représentent chacun un groupement éthylène (A et/ou B étant alors de préférence un groupement éthylène), ou bien les couples $R_1$, $R_3$ et $R_2$, $R_4$ forment avec A (ou avec B) et avec les atomes d'azote auxquels ils sont rattachés un groupement

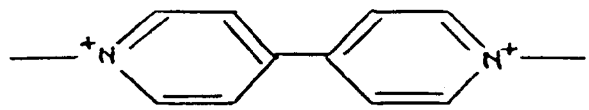

- A et/ou B représentent notamment un groupement alkylène ayant par exemple 2 à 12 atomes de carbone dans la chaîne, éventuellement substitué (par exemple par un ou plusieurs alkylène ayant 1 à 10 atomes de carbone, et/ou par un ou plusieurs groupements -OH ou =O) et/ou éventuellement interrompu par un ou plusieurs groupements arylène et/ou par un ou plusieurs hétérocycles et/ou par un ou plusieurs hétéroatomes ou groupements hétéroatomiques tels que -O-, -S-, -SO-, $-SO_2-$, -S-S-, $-N(R_5)-$ ou $-N^+(R_6)_2.X_1^-$,

$R_5$ étant un hydrogène ou un alkyle ayant par exemple 1 à 12 atomes de carbone, un aryle ayant 6 à 20 atomes de carbone ou un aralkyle (en particulier phénylalkyle) dont l'alkyle comporte 1 à 3 atomes de carbone, $R_5$ étant un alkyle ayant 1 à 10 atomes de carbone, et $X_1^-$ étant un anion; par exemple A et/ou B représentent un groupement

$$-(CH_2)_n-Z-(CH_2)_n-$$

où n représente un nombre entier de 1 à 10 et Z représente par exemple un groupement divalent de formule

$$-N(R_5)-CO-N(R_5)-$$

$$-N(R_5)-CO-Y_1-CO-N(R_5)$$

$$-N(R_5)-SO_2-(C_6H_4-C_6H_4-SO_2)_p-N(R_5)-$$

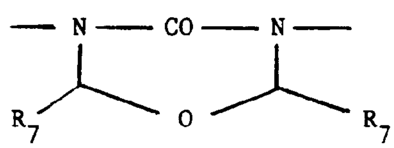 , 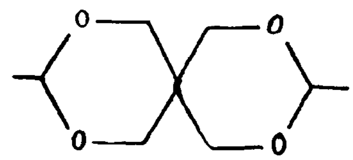

$$-CH(OH)-, -CO-N(R_5)-, -CO-O-, -O-CO-NH-, -CO-X_2-CO-$$

ou

$$-O-CO-X_3-CO-O-,$$

où $R_5$ est défini comme précédemment,

$R_7$ a la même signification que $R_5$,

$R_5$ représente H ou alkyle inférieur,

$Y_1$ représente un alkylène (éventuellement interrompu par un groupement -S-S-), un groupement alcénylène 4-20C, arylène, diaminoalkylène, diaminoarylène, dioxyalkylène, dioxyarylène, polyoxyalkylène, ou une liaison covalente directe,

p est un nombre égal à 0 ou 1,

$X_2$ est un groupement diamino-alkylène (à terminaisons amino), dioxyalkylène ou polyoxyalkylène (à terminaisons oxy), ou un groupement dithioalkylène (à terminaisons thio) dont les alkylène ont 2 à 20 atomes de carbone,

$X_3$ est un alkylène, cycloalkylène ou arylène, substitué ou non, ou un groupement diaminoalkylène, diaminocycloalkylène ou diaminoarylène;

- ou bien Z représente un groupement arylène ou plusieurs groupements arylène éventuellement reliés par un hétéroatome ou par un groupement hétéroatomique, Z étant par exemple un groupement $-C_6H_4-$, $-C_6H_4-C_6H_4-$, $-C_6H_4-O-C_6H_4-$, $-C_6H_4-SO_2-C_6H_4-$ou

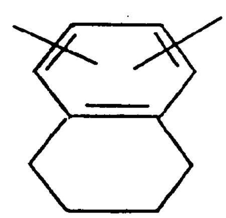

- ou bien A et/ou B représentent un groupement divalent $-B_1-Y_2-B_2-$, dans lequel $B_1$ et $B_2$ représentent des groupements arylène et $Y_2$ représente un groupement aliphatique divalent (par exemple un alkylène linéaire ou ramifié ayant 1 à 10 atomes de carbone) éventuellement substitué (par exemple par un ou plusieurs groupements -OH ou =O) et/ou interrompu par un ou plusieurs hétéroatomes et/ou par un ou plusieurs groupements hétéroatomiques et/ou par un ou plusieurs hétérocycles, ou $Y_2$ représente un hétéroatome ou un groupement d'hétéroatomes, ou $Y_2$ représente (indépendamment de Z) un groupement divalent choisi parmi ceux que peut représenter Z (à l'exception d'arylène); lorsque A ou B représente $-B_1-Y_2-B_2$, il peut s'agir par exemple d'un groupement $-C_6H_4-$ , $-C_6H_4-C(CH_3)_2-C_6H_4-$,$C_6$ $H_4-SO_2-C_6H_4-$, $C_6H_4-CO-C_6H_4-$ ou $-C_6H_4-CHOH-C_6H_4-$;
- ou bien A et/ou B représentent un groupement de formule

$$-(EO)_{m1}-(DO)_m-D_1-$$

E étant un groupement alkylène ayant 1 à 10 atomes de carbone ou un hydroxyalkylène ayant 1 à 10 atomes de carbone,

D étant un groupement hydrocarboné divalent ayant 1 à 10 atomes de carbone,

m1 étant un nombre égal à 0 ou 1,

m étant un nombre de 1 à 600,

$D_1$ étant identique à E quand $m_1$=1 et identique à D quand $m_1$=0;
- ou bien A et/ou B représentent un groupement

$$-E-O-G-O-E-,$$

E étant défini comme précédemment et G étant un groupement hydrocarboné, tel qu'un alkylène, cycloalkylène, arylène ou aralkylène, éventuellement substitué, notamment par un ou plusieurs groupements -OH et/ou =O ; E représente par exemple $-CH_2CH(OH)CH_2-$ou un alkylène ayant 1 à 4 C;
- l'un des substituants A ou B peut encore représenter, par exemple, un groupement de formule

$$-(CH_2)_{n'}-CO-X'_2-CO-(CH_2)_{n'}-$$

dans laquelle $X'_2$ représente :

a) un groupement

$$-O-(CH_2-CH_2-O)_x-CH_2-CH_2-O-$$

ou

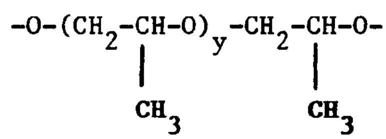

où x et y désignent un nombre entier de 1 à 4 représentant un degré de polymérisation défini ou un nombre quelconque de 1 à 4 représentant un degré de polymérisation moyen ;

b) un reste de diamine bis-secondaire tel qu'un reste dérivé de la pipérazine;

c) un reste de diamine bis-primaire de formule :

$$- NH - Y - NH -$$

où Y désigne un radical bivalent hydrocarboné ayant 2 à 20C, tel qu'un alkylène linéaire ou ramifié, ou le groupement bivalent

$$- CH_2 - CH_2 - S - S - CH_2 - CH_2 -;$$

ou

d) un groupement uréylène de formule - NH - CO - NH-; et n' désigne le nombre 1 ou un nombre entier de 3 à 10;

et dans ce cas l'autre substituant A ou B représente notamment un alkylène linéaire ou ramifié, éventuellement insaturé.

Dans la présente demande, on désigne par l'expression "alkyle (ou alkylène) inférieur" un alkyle (ou alkylène) ayant 1 à 6 C.

Des polymères de ce type sont décrits en particulier dans les brevets et demandes de brevets français 2.320.330, 2.270.846, 2.316.271, 2.232.563, 2.336.434, 2.389.374, 2.399.451, 2.413.907, 2.471.776, 2.471.777, 2.471.996, 2.471.997 et les brevets des EUA 2.261.002, 2.271.378, 2.273.780, 2.375.853, 2.388.614, 2.454.547, 3.206.462, 3.734.889, 3.874.870, 4.001.432, 3.929.990, 3.966.904, 4.005.193, 4.025.617, 4.025.627, 4.025.653, 4.026.945 et 4.027.020.

Les polymères polycationiques utilisables selon l'invention, y compris ceux contenant des motifs de formule I, peuvent aussi être des polymères séquencés.

Les polymères polycationiques azotés, tels que définis ci-dessus, qui sont utilisables selon la présente

invention, ont une masse moléculaire (déterminée par exemple par la méthode de diffusion de lumière au photogoniodiffusomètre : SICA ; appareil de Wippler et Scheibling) généralement supérieure à 1000. Il n'y a pas de valeur limite supérieure fixe de la masse moléculaire, la seule condition limite étant que le polymère (sous forme de fluorure) reste soluble dans l'eau. A titre d'exemple, on peut indiquer que les polymères utilisables selon l'invention ont généralement une masse moléculaire pouvant varier de 1000 à 100.000 environ.

Parmi les polymères polycationiques actuellement préférés, on citera par exemple ceux dont les motifs de formule I présentent au moins l'une des caractéristiques non mutuellement exclusives suivantes :

- $R_1$, $R_2$, $R_3$, $R_4$ sont des groupements aliphatiques saturés ayant 1-12 atomes de carbone ;
- $R_1$, $R_2$, $R_3$, $R_4$ sont des groupements alkyle ayant 1-12 atomes de carbone ;
- $R_1 = R_3 = CH_3$ ;
- $R_1 = R_3 = R_4 = CH_3$ ;
- A et B représentent un alkylène linéaire ou ramifié ayant 3 à 10 atomes de carbone dans la chaîne ;
- A représente un groupement alkylèneoxyalkylène ayant 4 à 12 atomes de carbone,

  et B représente :

$$-(CH_2)_n\text{-NH-CO-NH-}(CH_2)_n$$

ou

$$-(CH_2)_n\text{-NH-CO-Alk-CO-NH-}(CH_2)_n\text{-},$$

Alk représentant un alkylène ayant 1 à 34 atomes de carbone et n étant un nombre entier de 1 à 10 ;

- $R_1 = R_2 = R_3 = R_4 = CH_3$,

  A représente éthylèneoxyéthylène

  et B représente :

  soit

$$-(CH_2)_3\text{-NH-CO-NH-}(CH_2)_3\text{-},$$

  soit

$$-(CH_2)_3\text{-NHCO-}(CH_2)_4\text{-CONH-}(CH_2)_3\text{-},$$

  soit

$$-(CH_2)_3\text{-NHCO}(CH_2)_7\text{-CONH-}(CH_2)_3\text{-}.$$

Comme indiqué ci-dessus, les compositions dentifrices de l'invention peuvent contenir en outre tous les ingrédients habituels utilisés dans ce type de produits. Elles sont préparées selon les procédés usuels. Elles peuvent contenir en particulier au moins un agent de polissage, par exemple minéral. Elles peuvent également contenir un agent tensio-actif.

Les agents tensio-actifs utilisables peuvent être de nature anionique, amphotère, cationique, ou non-ionique. De préférence, on utilise des tensio-actifs cationiques ou des tensio-actifs non-ioniques.

Parmi les tensio-actifs cationiques on citera les composés alkylammonium (hétérocycles saturés ou non), les alkénylamines et alkyl amines primaires, les alkyl amines secondaires ou tertiaires, les amines tertiaires, les éthers d'amines, et les alkylènediamines primaires, secondaires ou tertiaires.

Parmi les tensio-actifs non-ioniques on citera les alkylphénols polyoxyéthylénés, les alcools polyoxyéthylénés, les esters polyoxyéthylénés d'acides gras, les alkylamines polyoxyéthylénées, les alkylamides polyoxyéthylénés, les esters de glycols ou de glycérol, les esters polyglycérolés, les esters de tétritol, de pentritol, d'hexitol ou d'anhydrohexitol, les esters de sucres, les esters de polyol polyoxyalkylénés, et les copolymères à motifs polyalkylène-oxyde (en particulier, TERGITOLS et PLURONICS).

On peut utiliser en particulier les tensio-actifs non ioniques poly(hydroxypropyléther) choisis parmi les composés de formules (I), (II) et (III) ci-après et/ou parmi les composés préparés selon le procédé décrit dans le paragraphe (iv) ci-dessous :

(i)

$$R'_6O - (CH_2 - \underset{\substack{|\\ CH_2OH}}{CHO})_z\text{-H} \qquad (I)$$

où $R'_6$ désigne un groupement ou un mélange de groupements alkyle contenant 10 à 14 atomes de carbone et z est un nombre entier ou décimal de 2 à 10 et de préférence de 3 à 6;

(ii)

$$R'_7\text{-CHOH - }CH_2\text{-O - }(CH_2\text{ - CHOH - }CH_2O)_z\text{-H} \qquad (II)$$

où $R'_7$ désigne un groupement ou un mélange de groupements alkyle ayant de 8 à 12 atomes de carbone et z' désigne un nombre entier ou décimal de 2 à 10 et de préférence de 2,5 à 6;

(iii)

$$R''_1\text{-CONH-CH}_2\text{-CH}_2\text{-O-CH}_2\text{-CH}_2\text{-O-(CH}_2\text{-CHOH-CH}_2\text{-O)}_q\text{-H} \qquad \text{(III)}$$

où $R''_1$ désigne un groupement ou un mélange de groupements alkyle et/ou alkényle ayant de 11 à 18 atomes de carbone, et q désigne un nombre entier ou décimal de 1 à 5 et de préférence de 1,5 à 4;

(iv) les composés préparés par condensation, en catalyse acide, de 2 à 10 et de préférence de 2,5 à 6 moles de glycidol par mole d'alcool ou d'alcane- diol-1,2 contenant 10 à 14 atomes de carbone. Le procédé de préparation de ces composés est décrit dans le brevet français 2.169.787.

Ces tensio-actifs non ioniques particuliers sont décrits notamment dans les demandes de brevets français 1.477.048, 2.091.516 et 2.328.763.

Les agents tensio-actifs de l'invention sont présents dans une gamme variant de 0 à 20% en poids par rapport au poids total de la composition et généralement, lorsqu'ils sont présents, dans la gamme 0,5 à 5%.

L'invention concerne également une composition dentifrice, telle que définie précédemment, ne contenant pas d'agent tensio-actif cationique. Une telle composition contient par exemple, comme agent tensio-actif, au moins un tensio-actif non ionique tel que défini précédemment.

Comme indiqué ci-dessus, les compositions de l'invention peuvent contenir un agent de polissage. L'invention a en particulier pour objet de telles compositions dentifrices contenant un agent de polissage, notamment un agent de polissage minéral. Les agents de polissage sont des agents abrasifs compatibles avec l'utilisation dans les compositions dentifrice. Des exemples de tels agents de polissage seront donnés ci-après.

Comme cela a été indiqué ci-dessus, les fluorures de polymères polycationiques utilisés selon la présente invention possèdent une activité antibactérienne. On peut toutefois ajouter dans la composition dentifrice d'autres composés cationiques à caractère antibactérien, tels que par exemple le chlorure de diisobutyl phénoxyéthoxy éthyl diméthyl benzyl ammonium (Hyamine 1622), le bromure de dodécyl triméthyl ammonium, le bromure de dodécyl diméthyl (2-phénoxyéthyl) ammonium, le chlorure de benzyl diméthyl stéaryl ammonium, le chlorure de cétyl pyridinium, la 5-amino 1,3 bis (2-éthyl hexyl) - 5 méthyl hexahydroxypyrimidine quaternisée, le bromure de triméthyl cétyl ammonium, le bromure d'alkyl diméthyl hydroxyéthyl ammonium (avec alkyl = reste coprah), la chlorhexidine, l'alexidine, les amines tertiaires aliphatiques cationiques.

Ces agents antibactériens complémentaires, lorsqu'ils sont présents, peuvent représenter jusqu'à 10% du poids total de la composition dentifrice. De préférence on utilisera ces composés à des concentrations comprises entre 0,05 et 2%.

L'invention concerne notamment des compositions dentifrices, telles que définies ci-dessus, ne contenant pas d'agents antibactériens cationiques complémentaires, et en particulier des compositions dentifrices qui, en outre, ne contiennent pas d'agent tensio-actif cationique ; et, parmi ces compositions, celles qui contiennent un agent de polissage, notamment un agent de polissage minéral.

Les compositions dentifrices selon l'invention se présentent sous forme de pâtes, de gels, de gommes à mâcher, de poudres ou de bains de bouche.

Les pâtes contiennent généralement un matériau de polissage abrasif minéral constitué par un ou plusieurs composés en grande partie insolubles dans l'eau; on citera par exemple les métaphosphates de sodium ou de potassium, le phosphate de calcium dihydraté, le phosphate dicalcique, le phosphate tricalcique, le pyrophosphate de calcium, l'alumine, les alumines hydratées et, en particulier trihydratées, la silice, les silicates d'aluminium ou de zirconium, la bentonite, ainsi que l'orthophosphate de magnésium ou le phosphate trimagnésien.

Dans le cas de gels transparents on utilisera par exemple un agent de polissage à base de silice colloïdale ou d'aluminosilicates de métaux alcalins ou alcalinoterreux, de préférence de sodium ou de calcium.

Les gommes à mâcher contiennent principalement, outre les ingrédients actifs, au moins une gomme masticable naturelle ou synthétique, des agents d'aromatisation, des agents sucrants, humectants, des bactéricides et des conservateurs.

Parmi les gommes ayant une plasticité suffisante (seules ou en mélange) pour être masticables, on citera la carboxyméthylcellulose sodique et la gomme tragacanth.

Généralement les gommes à mâcher contiennent de 0,5 à 70% en poids de gomme masticable.

Les agents de polissage utilisables dans les gommes à mâcher peuvent être d'origine minérale ou organique. Ce sont par exemple les carbonates de calcium, magnésium, sodium, les phosphates et sulfates de calcium, l'alumine et l'alumine hydratée, la silice, les oxyde, hydroxyde, trisilicate et pyrophosphate de magnésium, ou des composés cellulosiques obtenus par broyage de graines de céréales.

Les agents de polissage représentent 10 à 80%, et de préférence 15 à 65 %, du poids total de la composition.

On peut également introduire dans les compositions dentifrices selon l'invention, des agents de cohésion

qui peuvent être des gommes naturelles ou des épaississants synthétiques.

Comme gommes naturelles on peut citer la gomme adragante, les gommes de xanthane, les gommes de guar, de caroube ou de carraghénanes.

Comme épaississants synthétiques on utilise essentiellement des dérivés de cellulose comme le sel de sodium de la carboxyméthyl cellulose, la méthylcellulose ou les hydroxyalkylcelluloses.

Ces agents de cohésion, lorsqu'ils sont présents dans les compositions dentifrices selon l'invention, sont généralement incorporés dans une proportion pondérale pouvant aller jusqu'à 10% et de préférence entre 0,5 et 3%.

Les compositions sous forme de bains de bouche sont des compositions dentifrices liquides, qui contiennent essentiellement une solution aqueuse d'un agent moussant, et éventuellement un agent épaississant, des agents bactéricides, des édulcorants et des substances aromatisantes. Ces compositions liquides peuvent contenir en outre de petites quantités d'un agent de polissage ultra-fin.

Les compositions dentifrices sous forme de poudres contiennent essentiellement un agent de polissage et un agent moussant.

En général, on ajoute également dans les compositions dentifrices un agent édulcorant, comme par exemple le saccharose, le lactose, le fructose, le xylitol, le cyclamate de sodium, le maltose ou le saccharinate de sodium, à des concentrations allant généralement jusqu'à 2%, et variant par exemple entre 0,1 et 2% du poids total de la composition.

Pour assurer une bonne pureté bactériologique des formulations selon l'invention, il est encore possible d'utiliser un agent conservateur comme ceux qui sont couramment utilisés dans ce genre de produits : formol et ses dérivés, parahydroxybenzoate de méthyle, parahydroxybenzoate de propyle, etc... Ces produits peuvent être présents dans un domaine de concentrations allant jusqu'à 0,5% et variant par exemple de 0,01 à 0,5% en poids.

La plupart du temps, on ajoutera à la composition dentifrice une substance aromatisante dans une proportion pouvant aller généralement jusqu'à 5%, et variant par exemple entre 0,5 et 5% par rapport au poids total de la composition : essences de menthe, d'anis, d'eucalyptus, de cannelle, de girofle, de sauge, de réglisse, essences de fruits tels que le citron, l'orange, la mandarine et la fraise, ou, éventuellement, le salicylate de méthyle.

Le pH de la composition dentifrice mesuré de manière conventionnelle pour une dispersion à 20% de pâte dans l'eau est habituellement compris entre 4,5 et 9.

La zone préférentielle de pH se situe entre 5,5 et 8,5.

Les pH alcalins ne sont généralement utilisés que dans le cas de matières premières instables en milieu neutre ou acide. C'est par exemple le cas des pâtes dentifrices utilisant le carbonate de calcium comme agent de polissage.

Dans la plupart des cas, on pourra ajuster le pH à l'aide d'un agent modificateur de pH tel que par exemple l'acide citrique, l'acide benzoïque, ou les phosphates monosodique et disodique.

L'invention a également pour objet l'utilisation, dans des compositions dentifrices contenant des agents de polissage minéraux et des agents tensio-actifs, de fluorures de polymères polycationiques comme agents anti-caries.

Les compositions dentifrices de l'invention sont appliquées chez l'homme selon les méthodes usuelles.

Les exemples suivants illustrent l'invention sans toutefois la limiter. Dans ces exemples, on désigne par:

- fluorure polycationique A : un ionène à base de motifs de formule I, avec $R_1=R_2=R_3=R_4=CH_3$, $A=(CH_2)_6$, $B=(CH_2)_3$,
  tel que 90% des $X^-$ sont des ions fluorure et 10% des ions chlorure ;
- composé B : le composé SATIAGUM VZ 40 (commercialisé par CECA-SATIA) qui est un complexe de carraghénane et d'alginate,
- composé C : le composé de formule :

$$R\text{-}CHOH\text{-}CH_2O - [CH_2\text{-}CHOH\text{-}CH_2O]\text{-}_nH$$

dans laquelle R = alkyle $C_{9-12}$

$$n = 3,4$$

- fluorure polycationique D : un ionène à base de motifs de formule I, avec $R_1=R_3=CH_3$, $R_2=R_4=C_3H_7$, $A=(CH_2)_3$,
  $B=(CH_2)_4$, tel que 80% des $X^-$ sont des ions fluorure et 20% des ions chlorure ;
- composé E : le composé SATIAGUM HE 41 (commercialisé par CECA- SATIA) qui est un complexe carraghénane/alginate
- composé F : le composé VEEGUM (commercialisé par Vanderbilt) qui est un silicate de magnésium et d'aluminium

- composé G : le composé de formule :

$$C_{12}H_{25}O[C_2H_3(CH_2OH)\text{-}O]_4H$$

- fluorure polycationique H : ionène à base de motifs de formule I, avec $R_1=R_2=R_3=R_4=CH_3$, $A=B=(CH_2)_{10}$, tel que 100% des $X^-$ sont des ions fluorure ;
- fluorure polycationique J : ionène à base de motifs de formule I, avec $R_1=R_3=CH_3$, $R_2=R_4=C_4H_9$, $A=B=(CH_2)_{10}$, tel que 5% des $X^-$ sont des ions fluorure, le reste étant des chlorures ;
- fluorure polycationique K : ionène à base de motifs de formule I, avec $R_1=R_3=CH_3$, $R_2=R_4=C_6H_{13}$, $A=B=(CH_2)_{10}$, tel que 50% des $X^-$ sont des ions fluorure, les 50% restants étant des chlorures ;
- fluorure polycationique L : ionène ayant des motifs de formule I, avec $R_1=R_3=CH_3$, $R_2=R_4=C_8H_{17}$, $A=B=(CH_2)_{10}$, tel que 80% des $X^-$ sont des ions fluorures, les 20% restants étant des chlorures ;
- fluorure polycationique M : ionène ayant des motifs de formule I, avec $R_1=R_3=CH_3$, $R_2=R_4=C_{12}H_{25}$, $A=B=(CH_2)_{10}$, tel que 90% des $X^-$ sont des ions fluorure, le reste étant des chlorures.

EXEMPLE 1

On a préparé une pâte dentifrice ayant la composition suivante :

| | | |
|---|---|---|
| - Fluorure polycationique A | 1,0 | g |
| - Alumine trihydratée | 55 | g |
| - Glycérine | 20 | g |
| - Composé B | 0,8 | g |
| - Composé C | 1 | g |
| - Arôme mentholé | 0,9 | g |
| - Eau | qsp.100 | g |

On a également préparé une pâte dentifrice de composition semblable, mais en remplaçant le fluorure polycationique A par le fluorure polycationique H.

EXEMPLE 2

On a préparé une pâte dentifrice ayant la composition suivante :

- Fluorure polycationique A................ 0,8 g
- Fluorure polycationique D................ 0,2 g
- Tensio-actif non ionique*................ 2 g
- Ethanol.................................. 3 g
- Sorbitol................................. 23 g
- Dihydrate de phosphate dicalcique......... 45 g

- Composé E................................ 0,4 g
- 1-6 di(4-chlorophényl biguanido) hexane gluconate......................... 0,3 g
- Arôme de fraise......................... 1 g
- eau................................qsp.100 g

* Copolymère polyoxyéthylène-polyoxybutylène dont le PM du résidu polyoxybutylène est de 1200 avec un contenu en polyoxyéthylène d'environ 80%.

On a préparé une pâte dentifrice analogue en remplaçant le fluorure polycationique A par le fluorure polycationique J.

EXEMPLE 3

On a préparé une pâte dentifrice de composition suivante :

- Fluorure polycationique A................ 0,5 g
- Monofluorophosphate de sodium............ 0,3 g
- Amido lauryl bétaïne d'acides gras de coprah ..................................1,7 g
- Hydroxyde d'aluminium microcristallin.... 43,5 g
- Glycérine................................ 27 g
- Propylène glycol......................... 1,5 g
- Gomme adragante.......................... 0,5 g
- Arôme d'anis............................. 1,1 g

- Phosphate monosodique.............qs pH7
- Eau.................................qsp.100 g

On a également préparé une pâte dentifrice analogue en remplaçant le fluorure polycationique A par le fluorure polycationique K.

EXEMPLE 4

On a préparé une pâte dentifrice de composition suivante :

- Fluorure polycationique D................ 1 g
- Silice micronisée........................ 25 g
- Glycérine................................ 25 g
- Gomme de xanthane........................ 1,3 g
- Arôme de réglisse........................ 1,2 g
- Saccharine............................... 0,15g
- Monolaurate de polyoxyéthylène sorbitan.. 2 g
- Hexachlorophène.......................... 0,03g
- Eau..................................qsp. 100 g

On a également préparé une pâte dentifrice analogue en remplaçant le fluorure polycationique D par le fluorure polycationique L.

EXEMPLE 5

On a préparé une pâte dentifrice de composition suivante :

- Fluorure polycationique A................ 0,4 g
- Fluorure de sodium....................... 0,1 g
- Dihydrate de phosphate dicalcique........ 33 g
- Phosphate dicalcique..................... 10 g
- Carbonate de calcium..................... 4 g
- Glycérine................................ 23 g
- Saccharine............................... 0,15g
- Composé B................................ 0,8 g
- Composé C................................ 1,3 g
- Composé F................................ 0,5 g
- Essence de citron........................ 1,3 g
- Eau..................................qsp. 100 g

On a préparé une pâte dentifrice analogue en remplaçant le fluorure polycationique A par le fluorure polycationique M.

EXEMPLE 6

On a préparé une composition dentifrice liquide de composition suivante :

- Fluorure polycationique D................ 0,4 g
- Saccharine............................... 0,03g
- Ethanol.................................. 25 g
- Glycérine................................ 10 g
- Composé G................................ 1 g
- Eau..................................qsp. 100 g

On a préparé une composition dentifrice liquide analogue en remplaçant le fluorure polycationique D par le fluorure polycationique L.

## Revendications

1. Composition dentifrice, caractérisée par le fait qu'elle contient, à titre d'agent anti-caries, au moins un polymère polycationique soluble dans l'eau, comprenant des motifs qui contiennent au moins un azote quaternisé faisant partie de la macrochaîne, et dont au moins 5% des charges cationiques sont équilibrées par des anions fluorure.

2. Composition selon la revendication 1, caractérisée par le fait que d'une part chaque atome d'azote quaternisé de la macrochaîne possède deux substituants latéraux choisis parmi des groupements aliphatiques, hydroxyaliphatiques, alicycliques ou arylaliphatiques, substitués ou non, contenant au maximum 20 atomes de carbone, ou bien les deux substituants latéraux attachés à un même atome d'azote constituent ensemble, avec celui-ci, un cycle pouvant contenir un second hétéroatome autre que l'azote, ou bien encore deux substituants latéraux attachés chacun à l'un de deux atomes d'azote consécutifs représentant ensemble un groupement divalent, et que d'autre part deux atomes d'azote quaternisés consécutifs sont reliés par un groupement divalent formant la macrochaîne.

3. Composition selon la revendication 2, caractérisée par le fait que ledit groupement divalent formant la macrochaîne est un groupement alcoylène ou arylène éventuellement substitué et pouvant comporter des hétéroatomes.

4. Composition selon la revendication 3, caractérisée par le fait que la macrochaîne est constituée par la succession de deux desdits groupements divalents, présents alternativement dans la chaîne, chacun entre deux atomes d'azote quaternisé consécutifs, ces deux groupements divalents pouvant être éventuellement identiques.

5. Composition selon l'une quelconque des revendications 1 à 4, caractérisée par le fait que ledit polymère polycationique est un ionène.

6. Composition selon l'une quelconque des revendications précédentes, caractérisée par le fait que ledit polymère polycationique comprend ou est constitué par des motifs de formule I

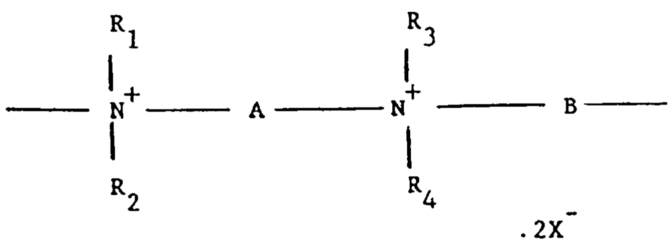

(I)

dans laquelle :

$R_1$, $R_2$, $R_3$ et $R_4$, identiques ou différents, représentent un groupement aliphatique, alicyclique ou arylaliphatique, éventuellement substitué, contenant au maximum 20 atomes de carbone, ou un groupement hydroxyaliphatique ayant 1 à 8 atomes de carbone; ou bien deux substituants ($R_1$ et $R_2$) et/ou ($R_3$ et $R_4$) attachés à un même atome d'azote peuvent représenter ensemble un groupement divalent formant, avec l'atome d'azote auquel ils sont rattachés, un hétérocycle pouvant contenir un ou plusieurs hétéroatomes autres que l'azote; ou bien les couples ($R_1$ et $R_3$) et/ou ($R_2$ et $R_4$) forment ensemble un groupement divalent reliant les deux atomes d'azote consécutifs, représentés dans le motif de formule I, auxquels ils sont respectivement rattachés ;

A et B, identiques ou différents, représentent un groupement alkylène linéaire ou ramifié ayant 2 à 20 atomes de carbone, éventuellement insaturé, ledit groupement alkylène pouvant être substitué et/ou interrompu par un ou plusieurs hétéroatomes, et/ou par un ou plusieurs groupements hétéroatomiques et/ou par un ou plusieurs cycles aliphatiques ou aromatiques ou par un ou plusieurs hétérocycles; un groupement cycloalkylène, éventuellement substitué et/ou comportant éventuellement des doubles liaisons, pouvant contenir jusqu'à 20 atomes de carbone; un ou plusieurs groupements arylène ayant 6 à 20 atomes de carbone, lesdits groupements arylènes étant éventuellement substitués, et/ou séparé par un ou plusieurs hétéroatomes et/ou par un ou plusieurs groupements hétéroatomiques et/ou par un ou plusieurs groupements alkylène et/ou par un ou plusieurs cycles aliphatiques et/ou par un ou plusieurs hétérocycles ;

et $X^-$ représente un anion, étant entendu que, dans une composition selon l'invention, au moins 5%, en nombre, des anions présents dans le polymère polycationique ayant des motifs de formule I sont des ions fluorure.

7. Composition selon la revendication 6, caractérisée par le fait que lesdits motifs de formule I présentent au moins l'une des caractéristiques non mutuellement exclusives suivantes :
   - $R_1$, $R_2$, $R_3$, $R_4$ sont des groupements aliphatiques saturés ayant 1-12 atomes de carbone ;
   - $R_1$, $R_2$, $R_3$, $R_4$ sont des groupements alkyle ayant 1-12 atomes de carbone ;
   - $R_1 = R_3 = CH_3$ ;
   - $R_1 = R_3 = R_4 = CH_3$ ;
   - A et B représentent un alkylène linéaire ou ramifié ayant 3 à 10 atomes de carbone dans la chaîne ;
   - A représente un groupement alkylèneoxyalkylène ayant 4 à 12 atomes de carbone

     et B représente :

$$-(CH_2)_n\text{-NH-CO-NH-}(CH_2)_n\text{-}$$

ou

$$-(CH_2)_n\text{-NH-CO-Alk-CO-NH-}(CH_2)_n\text{-},$$

Alk représentant un alkylène ayant 1 à 34 atomes de carbone,

et n étant un nombre entier de 1 à 10 ;

   - $R_1 = R_2 = R_3 = R_4 = CH_3$,

     A représente éthylèneoxyéthylène

     et B représente :

     soit

$$-(CH_2)_3\text{-NH-CO-NH-}(CH_2)_3\text{-},$$

soit

$$-(CH_2)_3\text{-NHCO-}(CH_2)_4\text{-CONH-}(CH_2)_3\text{-}$$

soit

$$-(CH_2)_3\text{-NHCO-}(CH_2)_7\text{-CONH-}(CH_2)_3\text{-}.$$

8. Composition selon l'une quelconque des revendications précédentes, caractérisée par le fait que la concentration en polymère polycationique est de 0,1 à 5% en poids par rapport au poids total de la composition.

9. Composition selon la revendication 7, caractérisée par le fait que ladite concentration est de 0,5 à 5%.

10. Composition selon l'une quelconque des revendications précédentes, contenant en outre un agent de polissage.

11. Composition selon l'une quelconque des revendications 1 à 9, contenant en outre un agent de polissage minéral.

12. Composition selon l'une quelconque des revendications précédentes, exempte d'agent tensio-actif cationique.

13. Composition selon l'une quelconque des revendications précédentes, exempte d'agents antibactériens cationiques complémentaires.

14. Utilisation, dans des compositions dentifrices, de fluorures de polymères cationiques comme agents anticaries, lesdits fluorures de polymères polycationiques étant tels que définis dans l'une quelconque des revendications 1 à 7.

15. Utilisation selon la revendication 14, dans laquelle la composition dentifrice contient un agent de polissage.

16. Utilisation selon la revendication 15, dans laquelle ledit agent de polissage est minéral.

17. Utilisation selon l'une quelconque des revendications 14 à 16, dans laquelle la composition est exempte d'agent tensio-actif cationique.

18. Utilisation selon l'une quelconque des revendications 14 à 17, dans laquelle la composition est exempte d'agents antibactériens cationiques complémentaires.

## Patentansprüche

1. Zahnpastazusammensetzung, dadurch gekennzeichnet, daß sie als Antikarieswirkstoff mindestens ein wasserlösliches polykationisches Polymer enthält, das Gegenstände enthält, die mindestens einen quaternären Stickstoff enthalten, der Teil der Makrokette ist, und worin mindestens 5 % der kationischen Ladungen durch Fluoride ausgeglichen sind.

2. Zusammensetzung nach Anspruch 1, dadurch gekennzeichnet, daß einerseits jedes quaternären Stickstoffatom der Makrokette zwei seitliche substituierte oder unsubstituierte Substituenten trägt, die aus aliphatischen, hydroxyaliphatischen, alicyclischen oder arylaliphatischen Gruppen mit maximal 20 Stickstoffatomen ausgewählt sind, oder die beiden seitlichen, an das gleiche Stickstoffatom gebundenen Substituenten bilden zusammengenommen mit diesem einen Ring, der ein zweites von Stickstoff verschiedenes Heteroatom enthalten kann, oder ferner stellen zwei seitliche Substituenten, wobei jeder an eines der zwei folgenden Stickstoffatome gebunden ist, zusammengenommen eine divalente Gruppe dar, und daß andererseits zwei aufeinander folgende quaternäre Stickstoffatome durch eine divalente Gruppe, die die Makrokette bildet, miteinander verbunden sind.

3. Zusammensetzung nach Anspruch 2, dadurch gekennzeichnet, daß die divalente Gruppe, die die Makrokette bildet, eine Alkylen- oder Arylengruppe ist, die gegebenenfalls substituiert ist und Heteroatome tragen kann.

4. Zusammensetzung nach Anspruch 3, dadurch gekennzeichnet, daß die Makrokette durch die Aufeinanderfolge der zwei vorgenannten divalenten Gruppen, die alternativ in der Kette vorhanden sind gebildet ist, wobei jede zwischen zwei aufeinanderfolgenden quaternären Stickstoffatomen verbunden ist, und wobei die beiden divalenten Gruppen gegebenenfalls identisch sein können.

5. Zusammensetzung nach einem der Ansprüche 1 bis 4, dadurch gekennzeichnet, daß das polykationische Polymer ein Ionen ist.

6. Zusammensetzung nach einem der vorhergehenden Ansprüche, dadurch gekennzeichnet, daß das vorgenannte polykationische Polymer Gegenstände der Formel I enthält oder durch sie gebildet wird:

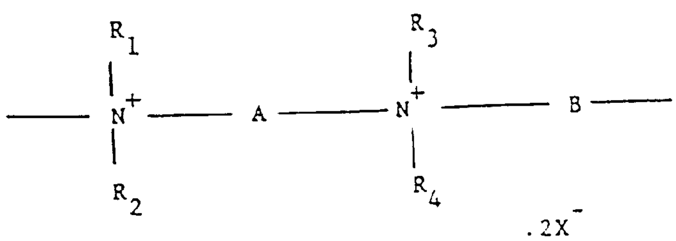

(I)

in der:
$R_1$, $R_2$, $R_3$ und $R_4$, die gleich oder verschieden sind, eine aliphatische, alicyclische oder arylaliphatische, gegebenenfalls substituierte Gruppe bedeuten, die maximal 20 Kohlenstoffatome enthält oder eine hydroxyaliphatische Gruppe mit 1 bis 8 Kohlenstoffatomen bedeuten; oder beide Substituenten ($R_1$ und $R_2$) und/oder ($R_3$ und $R_4$) die an ein gleiches Stickstoffatom gebunden sind, bedeuten zusammengenommen eine divalente Gruppe, die mit dem Stickstoff, an den sie gebunden sind, einen Heterocyclus bilden, der ein oder mehrere Heteroatome verschieden von Stickstoff tragen kann; oder die Paare ($R_1$ und $R_2$) und/oder ($R_3$ und $R_4$) bilden zusammengenommen eine divalente Gruppe, die die beiden aufeinanderfolgenden Stickstoffatome verbindet, wie in Formel I dargestellt, wo sie jeweils miteinander verbunden sind;

A und B, die gleich oder verschieden sind, eine lineare oder verzweigte, gegebenenfalls ungesättigte Alkylengruppe mit 2 bis 20 Kohlenstoffatomen bedeuten, worin die Alkylengruppe substituiert sein kann und/oder durch eines oder mehrere Heteroatome und/oder durch eine oder mehrere heteroatomhaltigen Gruppen und/oder durch eine oder mehrere aliphatische oder aromatische Ringe oder durch einen oder mehrere Heterocyclen unterbrochen sein kann bedeuten; eine gegebenenfalls substituierte Cycloalkylengruppe und/oder weist gegebenenfalls Doppelbindungen auf und enthält bis 20 Kohlenstoffatome bedeuten; eine oder mehrere Arylengruppen mit 6 bis 20 Kohlenstoffatomen, die gegebenenfalls substituiert sind und/oder durch eine oder mehrere Heteroatome und/oder durch eine oder mehrere Heteroatomgruppen und/oder durch eine oder mehrere Alkylengruppen und/oder einen oder mehrere aliphatischen Ringe und/oder durch eine oder mehrere heterocyclische Ringe getrennt sind, bedeuten;
und X ein Anion bedeutet, mit der Maßgabe, daß in der erfindungsgemäßen Zusammensetzung mindestens 5 %, als Zahl, der Anionen, die in dem polykationischen Polymer vorhanden sind, das Gegenstände der Formel I aufweist, Fluoridanionen sind.

7. Zusammensetzung nach Anspruch 6, dadurch gekennzeichnet, daß die Gegenstände der Formel I mindestens eine der folgenden charakteristischen Gruppen, nicht jedoch ausschließlich bedeuten:
   - $R_1$, $R_2$, $R_3$, $R_4$ sind gesättigte aliphatische Gruppen mit 1 bis 12 Kohlenstoffatomen;
   - $R_1$, $R_2$, $R_3$, $R_4$ sind Alkylgruppen mit 1 bis 12 Kohlenstoffatomen;
   - $R_1 = R_3 = CH_3$;
   - $R_1 = R_3 = R_4 = CH_3$;
   - A und B bedeuten ein verzweigtes oder lineares Alkylen mit 3 bis 10 Kohlenstoffatomen in der Kette;
   - A bedeutet eine Alkylenoxyalkylengruppe mit 4 bis 12 Kohlenstoffatomen
   und B bedeutet:

$$-(CH_2)_n-NH-CO-NH-(CH_2)_n-$$

   oder

$$-(CH_2)_n-NH-CO-Alk-CO-NH-(CH_2)_n-,$$

   worin Alk Alkylen mit 34 Kohlenstoffatomen bedeutet,
   und n bedeutet eine Zahl zwischen 1 und 10;
   - $R_1 = R_2 = R_3 = R_4 = CH_3$,

A bedeutet Ethylenoxyethylen
und B bedeutet:
entweder

$$-(CH_2)_3-NH-CO-NH-(CH_2)_3-,$$

entweder

$$-(CH_2)_3-NHCO-(CH_2)_4-CONH-(CH_2)_3-,$$

entweder

$$-(CH_2)_3-NHCO-(CH_2)_7-CONH-(CH_2)_3-.$$

8. Zusammensetzung nach einem der vorhergehenden Ansprüche, dadurch gekennzeichnet, daß die Konzentration des polykationischen Polymers 0,1 bis 5 Gew.-%, bezogen auf das Gewicht der Gesamtzusammensetzung beträgt.

9. Zusammensetzung nach Anspruch 1, dadurch gekennzeichnet, daß die Konzentration 0,5 bis 5 % beträgt.

10. Zusammensetzung nach einem der vorhergehenden Ansprüche, die außerdem ein Poliermittel enthält.

11. Zusammensetzung nach einem der Ansprüche 1 bis 9, die außerdem ein mineralisches Poliermittel enthält.

12. Zusammensetzung nach einem der vorhergehenden Ansprüche, die frei von kationischen Tensiden ist.

13. Zusammensetzung nach einem der vorhergehenden Ansprüche, die frei von kationischen antibakteriellen Ergänzungsmitteln ist.

14. Verwendung der kationischen polymeren Fluoride in einer Zahnpasta als Antikariesmittel, wobei die polykationischen Polymer Fluoride, die in den Ansprüchen 1 bis 7 gegebene Definition besitzen.

15. Verwendung nach Anspruch 14, in der die Zahnpasta ein Poliermittel enthält.

**16.** Verwendung nach Anspruch 15, in der das Poliermittel ein Mineral ist.

**17.** Verwendung nach einem der Ansprüche 14 bis 16, in der die Zusammensetzung frei von einem kationischen Tensid ist.

**18.** Verwendung nach einem der Ansprüche 14 bis 17, in der die Zusammensetzung frei von kationischen antibakteriellen Zusatzmitteln ist.

## Claims

**1.** Dentifrice composition characterised in that it contains, as anticaries agent, at least one water-soluble polycationic polymer comprising units which contain at least one quaternised nitrogen forming part of the macrochain, and in which at least 5 % of the cationic charges are equilibrated by fluoride anions.

**2.** Composition according to Claim 1, characterised in that, on the one hand, each quaternised nitrogen atom of the macrochain has two side substituents chosen from aliphatic, hydroxyaliphatic, alicyclic or arylaliphatic groups, which may be substituted, containing at most 20 carbon atoms, or else the two side substituents attached to the same nitrogen atom form together with the latter a ring which may contain a second heteroatom other than nitrogen, or else two further side substituents each of which is attached to one of two consecutive nitrogen atoms denoting together a divalent group, and in that, on the other hand, two consecutive quaternised nitrogen atoms are linked by a divalent group forming the macrochain.

**3.** Composition according to Claim 2, characterised in that the said divalent group forming the macrochain is an alkylene or arylene group which is optionally substituted and may contain heteroatoms.

**4.** Composition according to Claim 3, characterised in that the macrochain consists of the succession of two of the said divalent groups present alternately in the chain, each between two consecutive quaternised nitrogen atoms, it being optionally possible for these two divalent groups to be identical.

**5.** Composition according to any one of Claims 1 to 4, characterised in that the said polycationic polymer is an ionene.

**6.** Composition according to any one of the preceding claims, characterised in that the said polycationic polymer comprises or consists of units of formula I

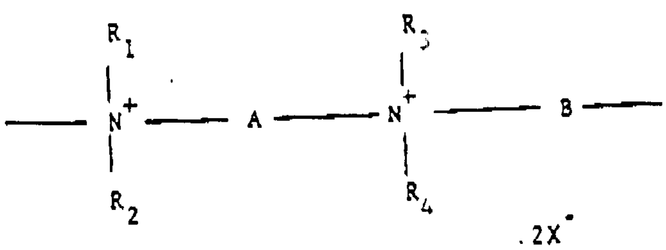

(I)

in which:

$R_1$, $R_2$, $R_3$ and $R_4$, which are identical of different, denote an aliphatic, alicyclic or arylaliphatic group which may substituted, containing at most 20 carbon atoms, or a hydroxyaliphatic group containing 1 to 8 carbon atoms; or else two substituents ($R_1$ and $R_2$) and/or ($R_5$ and $R_4$) attached to the same nitrogen atom may together denote a divalent group forming, with the nitrogen atom to which they are attached, a heterocyclic ring which may contain one or more heteroatoms other than nitrogen; or else the pairs ($R_1$ and $R_3$) and/or ($R_2$ and $R_4$) form together a divalent group linking the two consecutive nitrogen atoms, shown in the unit of formula I, to which they are respectively attached;

A and B, which are identical or different, denote an optionally unsaturated, linear or branched alkylene group containing 2 to 20 carbon atoms, it being possible for the said alkylene group to be substituted and/or interrupted by one or a number of heteroatoms and/or by one or a number of heteroatomic groups and/or by one or a number of aliphatic or aromatic rings or by one or a number of heterocyclic rings; a cycloalkylene group, optionally substituted and/or optionally containing double bonds, which may contain up to 20 carbon atoms; one or a number of arylene groups containing 6 to 20 carbon atoms, the

said arylene groups being optionally substituted, and/or which is separated by one or a number of heteroatoms and/or by one or a number of heteroatomic groups and/or by one or a number of alkylene groups and/or by one or a number of aliphatic rings and/or by one or a number of heterocyclic rings;

and $X^-$ denotes an anion, it being understood that in a composition according to the invention at least 5 % of the number of the anions present in the polycationic polymer containing units of formula I are fluoride ions.

**7.** Composition according to Claim 6, characterised in that the said units of formula I exhibit at least one of the following characteristics which are not mutually exclusive:

- $R_1$ $R_2$ $R_3$ and $R_4$ are saturated aliphatic groups containing 1-12 carbon atoms;
- $R_1$, $R_2$ $R_3$ and $R_4$ are alkyl groups containing 1-12 carbon atoms;
- $R_1 = R_3 = CH_3$;
- $R_1 = R_3 = R_4 = CH_3$;
- A and B denote a linear or branched alkylene containing 3 to 10 carbon atoms in the chain;
- A denotes an alkyleneoxyalkylene group containing 4 to 12 carbon atoms

and B denotes:

$$-(CH_2)_n-NH-CO-NH-(CH_2)_n-$$

or

$$-(CH_2)_n-NH-CO-Alk-CO-NH-(CH_2)_n-,$$

Alk denoting an alkylene containing 1 to 34 carbon atoms,

and n being an integer from 1 to 10;

- $R_1 = R_2 = R_3 = R_4 = CH_3$,

A denotes ethyleneoxyethylene

and B denotes:

either

$$-(CH_2)_3-NH-CO-NH-(CH_2)_3-,$$

or

$$-(CH_2)_3-NHCO-(CH_2)_4-CONH-(CH_2)_3-,$$

or

$$-(CH_2)_3-NHCO-(CH_2)_7-CONH-(CH_2)_3-.$$

**8.** Composition according to any one of the preceding claims, characterised in that the concentration of polycationic polymer is from 0.1 to 5 % by weight relative to the total weight of the composition.

**9.** Composition according to claim 7, characterised in that the said concentration is from 0.5 to 5 %.

**10.** Composition according to any one of the preceding claims, additionally containing a polishing agent.

**11.** Composition according to any one of Claims 1 to 9, additionally containing an inorganic polishing agent.

**12.** Composition according to any one of the preceding claims, free from any cationic surface-active agent.

**13.** Composition according to any one of the preceding claims, free from additional cationic antibacterial agents.

**14.** Use, in dentifrice compositions, of fluorides of cationic polymers as anticaries agents, the said fluorides of polycationic polymers being such as defined in any one of Claims 1 to 7.

**15.** Use according to Claim 14, in which the dentifrice composition contains a polishing agent.

**16.** Use according to Claim 15, in which the said polishing agent is inorganic.

**17.** Use according to any one of Claims 14 to 16, in which the composition is free from any cationic surface-active agent.

**18.** Use according to any one of Claims 14 to 17, in which the composition is free from additional cationic antibacterial agents.